# EUROPEAN PATENT SPECIFICATION

(11) **EP 3 638 262 B1**
(45) Date of publication and mention of the grant of the patent: **14.10.2026**
(21) Application number: 18817728.1
(22) Date of filing: 15.06.2018
(51) Int. Cl.: A61K 40/31, A61K 40/10, A61K 40/11, A61K 38/17, A61K 35/761, A61P 35/00, C07K 14/725, C12N 15/09, A61K 40/42

(54) **ACTIVATION OF T CELLS TO TARGET CANCER CELLS**
AKTIVIERUNG VON T-ZELLEN ZUR GEZIELTEN BEKÄMPFUNG VON KREBSZELLEN
ACTIVATION DES LYMPHOCYTES T POUR CIBLER LES CELLULES CANCÉREUSES

(30) Priority: 16.06.2017 US 201762521011 P; 17.01.2018 US 201862618399 P
(43) Date of publication of application: 22.04.2020
(73) Proprietor: Mayo Foundation for Medical Education and Research, Rochester, MN 55905 (US)
(72) Inventor: PEASE, Larry R., Rochester, Minnesota 55906 (US)
(74) Representative: Fish & Richardson P.C.
(86) International application number: PCT/US2018/037874
(87) International publication number: WO 2018/232318

(56) References cited:
- WO-A1-00/76320
- WO-A1-2013/063361
- WO-A1-2016/033331
- WO-A1-2017/075389
- WO-A2-2014/186469
- US-A1- 2008 226 664
- US-A1- 2012 141 537
- EDES I. ET AL.: "506. In Vivo Transduction of T Cells: The Future of Immunotherapy?", MOLECULAR THERAPY, vol. 24, no. 1, 1 May 2016 (2016-05-01), pages S201 - S202, XP055556364
- WAN ET AL.: "Probing the Effector and Suppressive Functions of Human T Cell Subsets Using Antigen-Specific Engineered T Cell Receptors", PLOS ONE, vol. 8, no. 2, 20 February 2013 (2013-02-20), pages 1 - 12, XP055556360

## Description

### CROSS-REFERENCE TO RELATED APPLICATIONS

This application claims priority to U.S. Application Serial Nos. 62/618,399, filed on January 17, 2018, and 62/521,011, filed on June 16, 2017.

### BACKGROUND

### 1. Technical Field

This document relates to materials and methods for activating naive T cells *in vivo.* For example, *in vivo* activation of naive T cells can be used to target cells (e.g., cancer cells) expressing a tumor antigen (e.g., a tumor-specific antigen).

### 2. Background Information

Approximately 22,000 people die from cancer each day globally. Cancers infiltrated by CD8+ T cells tend to have better prognoses than those devoid of these immune cells. However, effective antitumor cellular immunity is limited by the available T-cell receptor (TcR) repertoire consisting primarily of low affinity receptors specific for tumor associated antigens.

US 2012/141537 describes materials and methods for identifying and using MHC molecule variants for activating self-reactive T cells in a peptide-specific manner, and their use to focus autoimmune cellular responses against diseases such as cancers and persisting viral infections.

WO 2000/76320 describes a method of generating antigen specific allospecific, xenospecific, and allopeptide antigen specific human suppressor CD8+CD28- T cells, methods of treatment for reduction of risk of rejection of allografts and xenografts and autoimmune diseases using the human suppressor CD8+CD28- T cells.

WO 2017/075389 describes methods and composition for production of T cells and therapeutic methods using engineered T cells.

US 2008/226664 describes methods, compositions and kits for inducing and/or enhancing an immune response in a subject.

Edes, et al. (Molecular Therapy (2016)) describes *in vivo* transduction of T cells.

WO 2016/033331 describes a chimeric antigen receptor for binding with a target antigen and a method for generating the chimeric antigen receptor and cytotoxic cells that express the chimeric antigen receptor.

WO 2014/186469 describes methods and compositions for immunotherapy employing a modified T cell comprising a chimeric antigen receptor (CAR). The CAR-expressing T-cells can be produced using electroporation in conjunction with a transposon-based integration system to produce a population of CAR-expressing cells.

WO 2013/063361 describes a genetically modified non-human animal that includes in its genome unrearranged T cell receptor variable gene loci, as well as non-human embryos, cells, and tissues comprising the same, and constructs for making said genetically modified non-human animal.

Wan, et al. (PLoS One (2013)) describes probing the effector and suppressive functions of human T cell subsets using antigen-specific engineered T cell receptors.

### SUMMARY

The present invention is defined by the independent claims. The dependent claims depict other embodiments of the invention.

In accordance with the invention, provided herein is a nucleic acid encoding a heterologous antigen receptor, wherein said heterologous antigen receptor is a tumor-specific antigen receptor, and a replication-defective adenovirus including nucleic acid encoding an allogeneic MHC class I polypeptide, where the allogeneic MHC class I polypeptide is an HLA-A, HLA-B, or HLA-C polypeptide, for use in a method for obtaining an activated T cell to target cancer cells within a mammal and treating cancer within said mammal, where the activated T cell includes the heterologous antigen receptor. The method includes, or consists essentially of, administering to a mammal (a) the nucleic acid encoding a heterologous antigen receptor and (b) the adenovirus comprising nucleic acid encoding an allogeneic MHC class I polypeptide, where the nucleic acid is administered to the mammal after the adenovirus is administered to the mammal, and where the nucleic acid is administered to said mammal via injection into a lymph node of the mammal. The method includes activating T cells with the mammal, where administration of said adenovirus activates T cells within said mammal, where said adenovirus is administered to said mammal via intradermal injection , and where the nucleic acid is introduced into T cells within the mammal to form engineered T cells including the heterologous antigen receptor, where the nucleic acid encoding said heterologous antigen receptor is introduced into said T cells with a viral vector comprising said nucleic acid, wherein said viral vector is a lentiviral vector or retroviral vector, and where at least one T cell within the mammal includes the heterologous antigen receptor and is activated to target said cancer cells. The mammal can be a human. The at least one T can be a cytotoxic T lymphocyte. The antigen receptor can be a chimeric antigen receptor. The antigen receptor can be a tumor-specific antigen receptor. The at least one T cell can include a native T cell receptor. The at least one T cell can be a plurality of activated T cells including the heterologous antigen receptor. The activated T cells of the plurality of the activated T cells can include different native T cell receptors.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict between the present specification and a reference mentioned herein, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

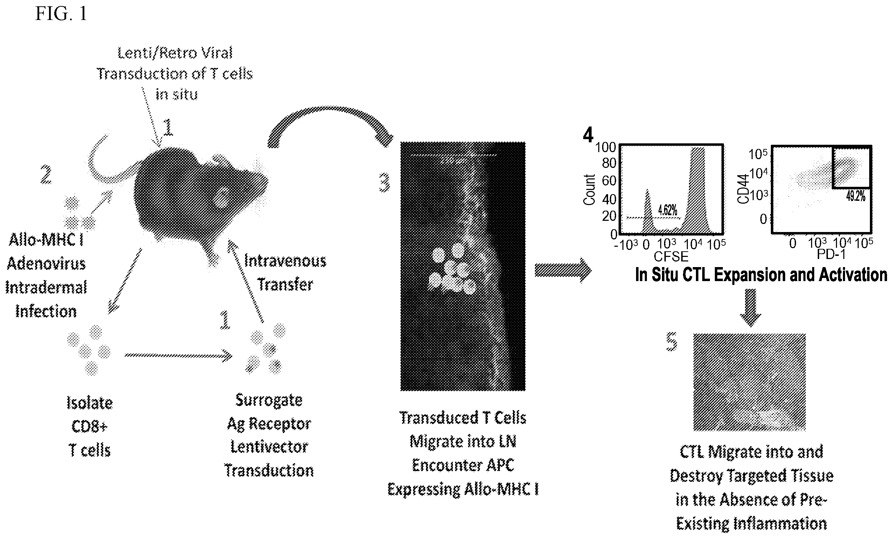

Figure 1 shows an exemplary scheme for *in vivo* activation of naive T cells expressing surrogate antigen receptors. (1) Isolated CD8+ T cells are transduced with lentivirus or retrovirus encoding surrogate receptors are adoptively transferred intravenously back into a host (bottom), or T cells are transduced *in situ* in draining lymph nodes (top). (2) Allogeneic MHC I (allo-MHC I) is expressed by adenovirus introduced intradermally. (3) Transduced T cells migrate into lymph nodes and encounter APC expressing allo-MHC I. (4) Allo-reactive CTLs are activated, and (5) leave lymph node and destroy cells expressing antigens targeted by surrogate receptors.

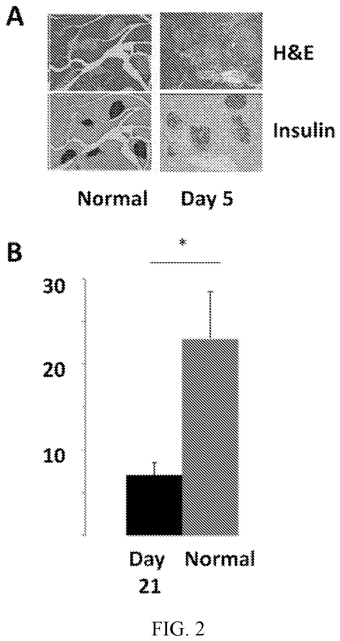

Figures 2A and 2B shows that normal tissue was targeted and destroyed by virus activated tissue-specific CTL. 1200 OT-1 T cells were adoptively transferred into RIP-OVA mice, then activated with TMEV-OVA. Figure 2A contains photographs of haemotoxylin and Eosin (H&E) staining and immunohistochemistry (IHC) staining for insulin showing pancreatic inflammation within 5 days of CTL induction by virus. Figure 2B contains a graph showing significant destruction of islets at day 21 in surviving mice. No virus was detected in pancreas by PCR. The pancreas was totally destroyed with increased numbers of OT-1 cells. Similar results were observed when replication defective adenovirus encoding ovalbumin was used to induce pancreas destruction by OT-1 T cells.

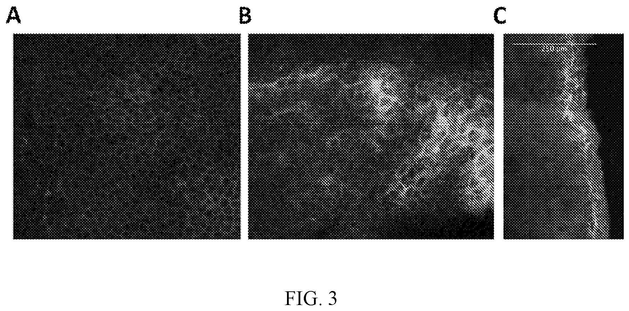

Figures 3A - 3C are photographs of fluorescent microscopy showing transduction of lymph node (LN) cells. mTmG-mice were infected by intradermal infection with an adenovirus expressing a cre recombinase (adeno-cre). Figure 3A shows that adeno control virus infected LN cells. Figure 3B shows that the adeno-cre infected LN and expressed cre recombinase in the LN. Figure 3C shows a low magnification view of LN showing marginal location of transduced cells.

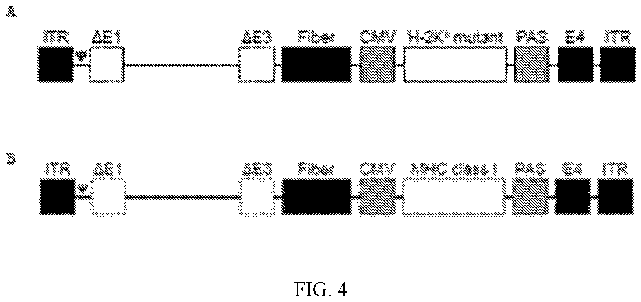

Figure 4A is a schematic of an exemplary replication-defective adenovirus (serotype 6) vector expressing a mutant MHC molecule, which functions as a universal alloantigen. Figure 4B is a generic version of the vector construct, by using an engineered mutant MHC molecule, the MHC can be universally allogeneic to any person. Alternatively, by using a naturally occurring MHC class I molecule, the MHC can be allogeneic to a cohort or subset of a population.

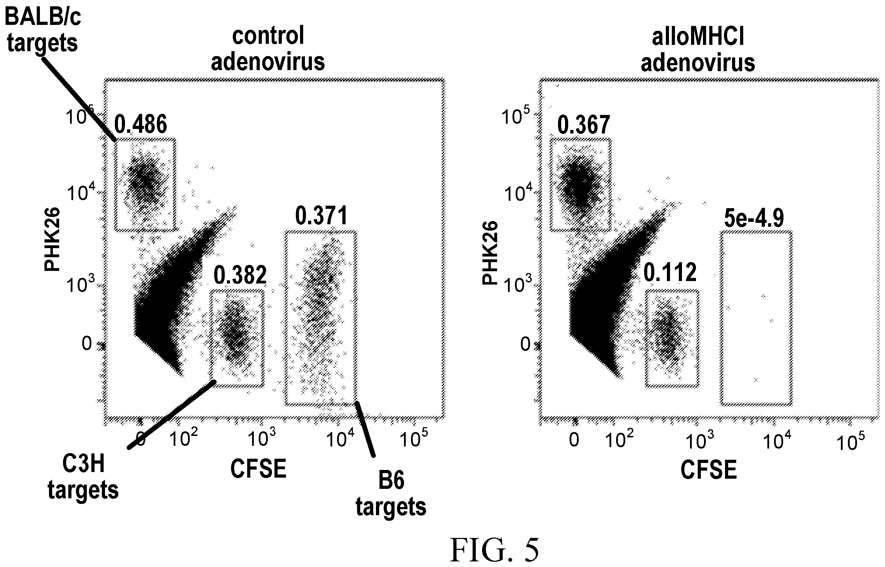

Figure 5 contains dot plots showing that allo-reactive CTLs were generated in response to adenovirus encoding allogeneic MHC I. Allo-MHC I adenovirus was introduced into LN by intradermal injection. Four days later, syngeneic (BALB/c), allogeneic (B6), and third party (C3H) labeled target cells were adoptively transferred intravenously into challenged hosts in an *in vivo* CTL assay. Four hours later spleen cells were harvested and analyzed by flow cytometry for the presence of introduced target cells. B6 target cells (targets expressing introduced allo-MHC I) were completely eliminated *in vivo.*

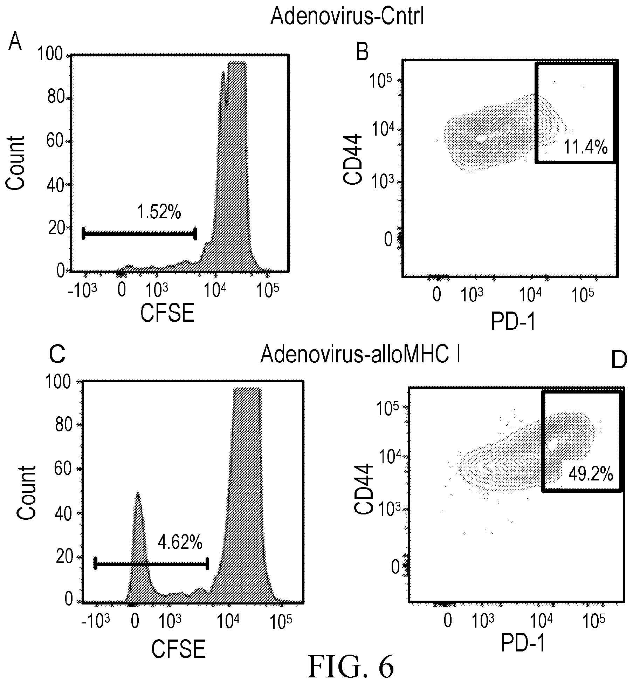

Figure 6 contains dot plots showing that adoptively transferred CD8+ T cells responded to adeno-alloMHCI. Freshly isolated syngenic CD8+ T cells were labeled with carboxyfluorescein succinimidyl ester (CFSE) before transfer, followed by challenge with adeno-allo-MHC I or control virus. Figures 6A and 6C show that adoptively transferred CFSE-labeled T cells migrate to the LN where they encounter and respond to transduced allo-MHC I molecules. Figure 6C also shows that the stimulated cells proliferate when stimulated with allo-MHC I, diluting the CFSE. Figures 6B and 6D shows that the CFSE-dilute population displayed a more activated phenotype expressing high CD44 and PD-1 (D) relative to the CFSE-dilute cells isolated from lymph nodes challenged with control adenovirus (B).

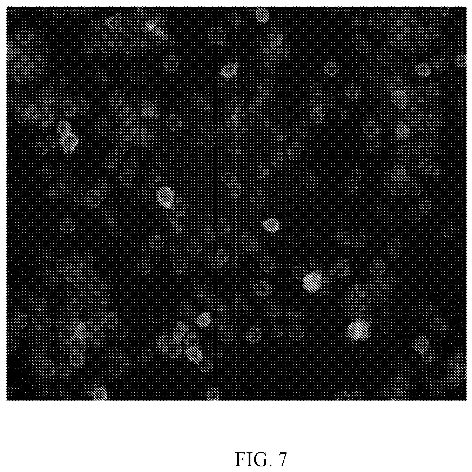

Figure 7 is a photograph of fluorescent microscopy showing lentivirus transduction of naive CD8+ spleen cells from a mTmG-reporter mouse. CD8+ enriched naive spleen cells were transduced with lentivirus-cre. The cells were subsequently activated with anti-CD3/CD28+ IL-2 to maintain viability in culture for 4 days. Successful transduction results in the transition from red to green fluorescence.

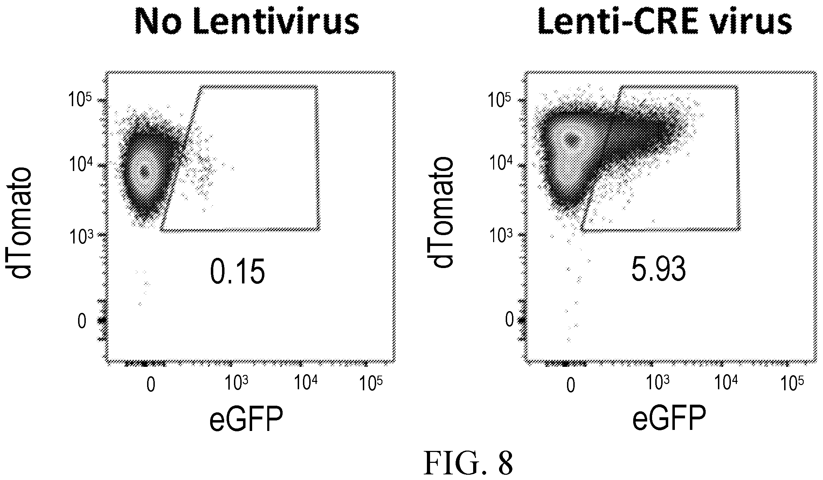

Figure 8 is a dot plot showing successful *in situ* introduction of transgene into activated lymph node cells. Adenoviral vector encoding alloMHC was injected intradermally into mTmG reporter mice to stimulate draining lymph node, four days later lentivirus-cre was directly injected into the enlarged lymph node. After 24 hours, CD8+ T cells from the lymph node were harvested and cultured for 3 days in the presence of IL2+IL7 to allow membrane eGFP expression.

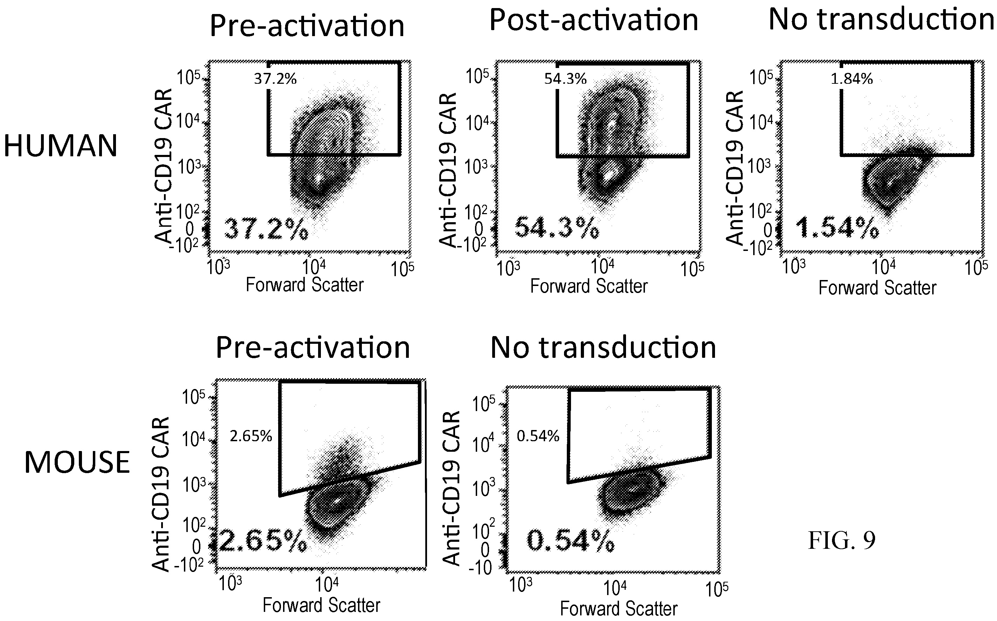

Figure 9 contains dot plots showing successful transduction of transgene into human cells. Human CAR lentiviral vector effective at transducing human, but not mouse T cells.

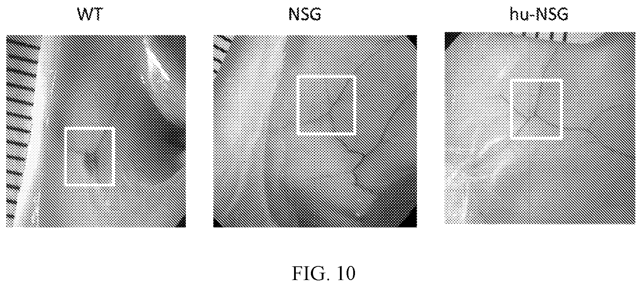

Figure 10 contains photographs showing intradermal introduction of non-replicating virus. Hu-NSG mice lack lymph nodes. Evans blue injected intradermally in the tail to mark inguinal lymph node in WT, NOD Scid IL-2Rγ^{-/-} (NSG), and human CD34+ hematopoietic cell reconstituted NSG mice (hu-NSG).

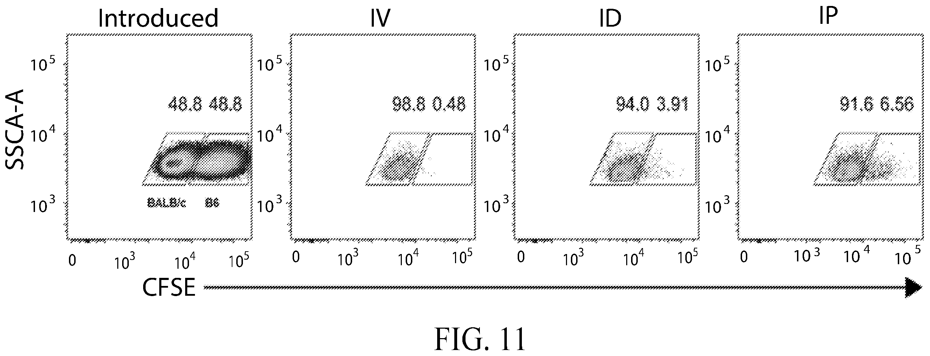

Figure 11 contains dot plots showing alternative routes of administration for in vivo CTL. All three immunization routes were effective as indicated by the relative depletion of the B6 target cells.

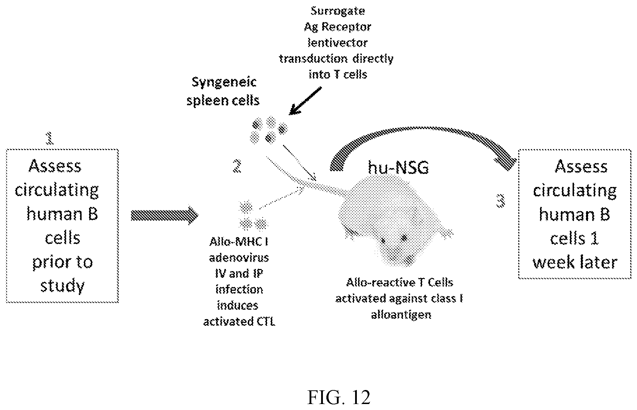

Figure 12 shows an exemplary scheme for using hu-NSG hosts. (1) Human B cells circulating in the hu-NSG host are assessed. (2) T cells from the spleen of the nu-NSG host are contacted with a lentivirus encoding a target antigen, and injected intravenously into the nu-NSG host mouse. Replication defective adenovirus 6 encoding the MHC allogeneic antigen H-2K^{b} are injected intravenously and an identical dose was injected intraperitoneally. (3) 1 week after treatment, the composition of human B cells in the blood is assessed.

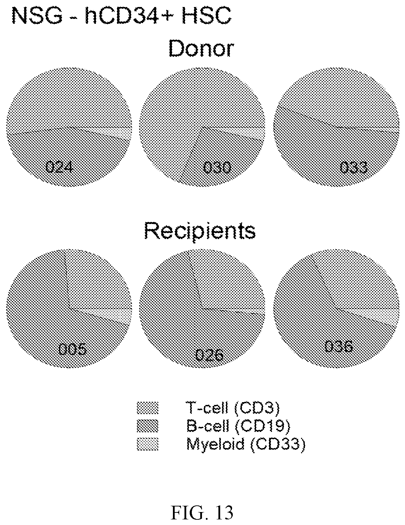

Figure 13 contains graphs showing human leukocyte composition prior to experiment of hu-NSG mice.

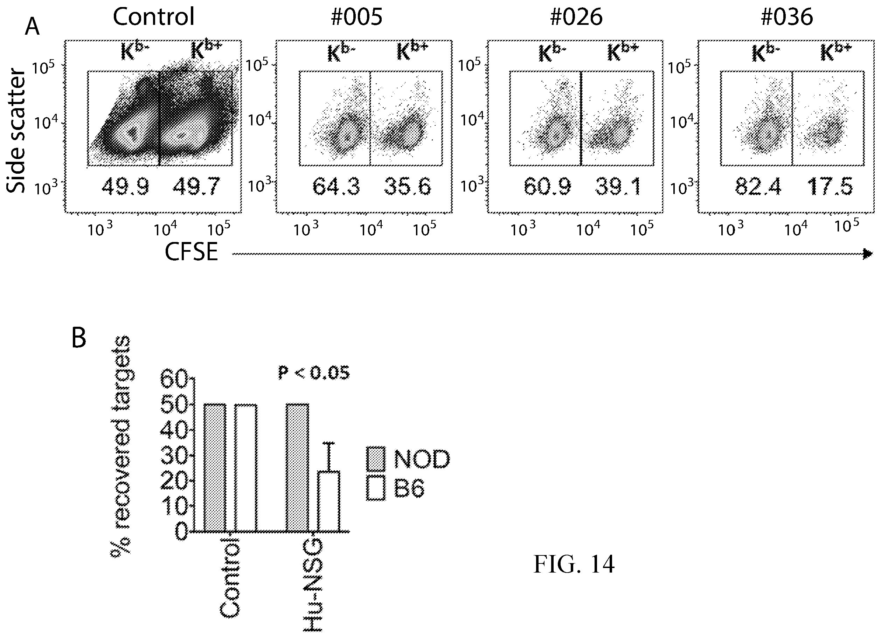

Figure 14 contains graphs showing *in vivo* CTL activates human immune cells in hu-NSG hosts. The expected 1: 1 ratio of recovered target cells was altered in all three recipients indicating a preferential killing of the K^{b+} spleen cells (panel A). The ratio of recovered K^{b+} cells was significantly lower relative to the K^{b-} target cells (panel B).

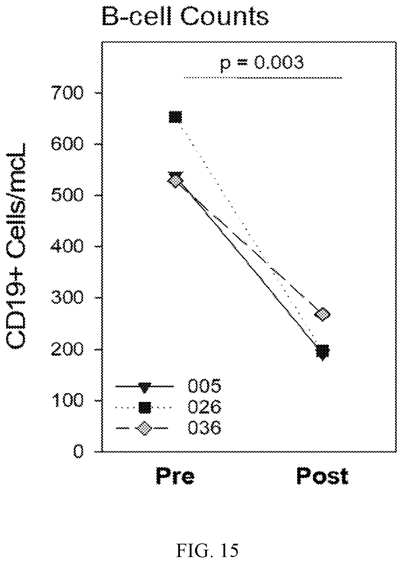

Figure 15 contains a graph showing raw data of the drop in B cell numbers in hu-NSG mice receiving CART treatment and AD6 vaccination.

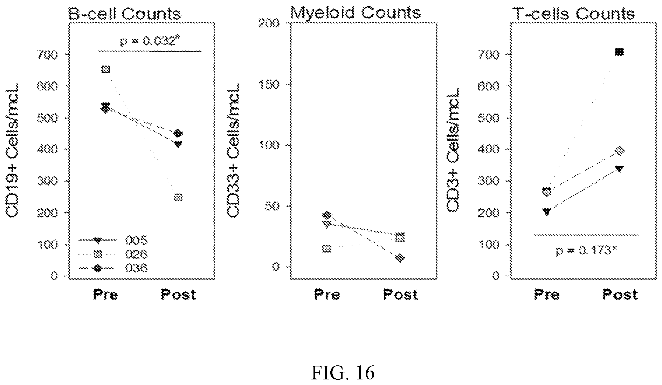

Figure 16 contains graphs showing normalized change in CD19+ B cells following introduction of Ad6-alloMHC (K^{b}) and lenti-CAR19 transduced spleen cells from hu-NSG mice reconstituted with CD34+ cells from the identical human donor. ^{a}Statistical evaluation normalized to account for the depletion of peripheral blood cell populations in the mice caused by repetitive blood sampling. *Increase in T cells following therapy is consistent with previous CART therapy findings.

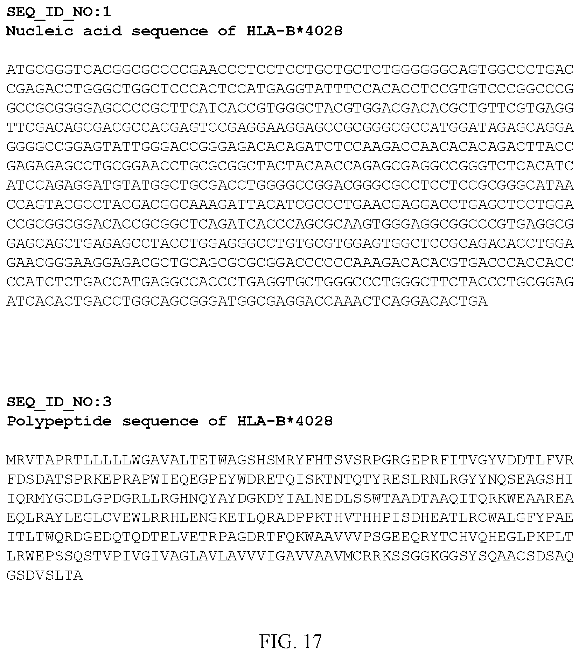

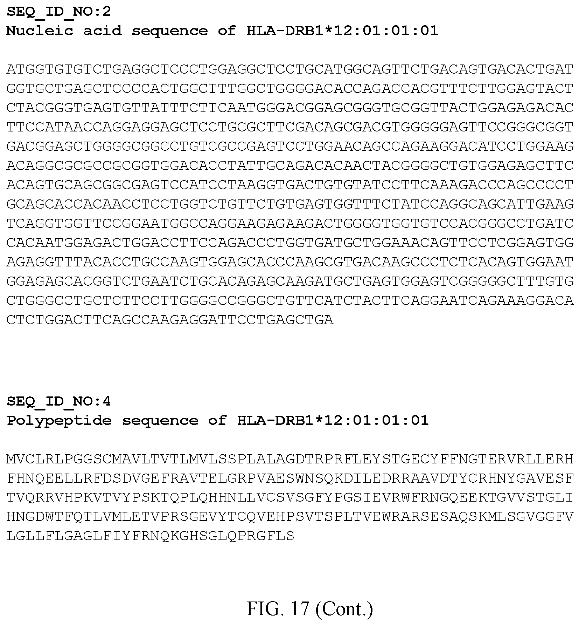

Figure 17 contains a sequence listing of a nucleic acid sequence (SEQ ID NO: 1) encoding a human MHC I polypeptide (an HLA-B40:28) and the amino acid sequence (SEQ ID NO:3) of that human MHC I polypeptide, and a sequence listing of a nucleic acid sequence (SEQ ID NO:2) encoding a human MHC I polypeptide (an HLA-DRB1*12:01:01:01) and the amino acid sequence (SEQ ID NO:4) of that human MHC I polypeptide.

### DETAILED DESCRIPTION

This document provides materials and methods for activating naive T cells (e.g., naïve T cells expressing tumor-specific antigen receptors) *in vivo* (e.g., making *in vivo* activated CTLs). For example, naive T cells expressing tumor-specific antigen receptors can be activated (e.g., to become CTLs) *in vivo* by encountering antigens (e.g., antigens presented on an APC such as a subcapsular sinus macrophage and/or a dendritic cell) in a lymph node. *In vivo* activated CTLs can include effector T cells and/or memory T cells. In some cases, naive T cells can be engineered to express tumor-specific antigen receptors *ex vivo.* For example, naive T cells can be obtained, engineered *ex vivo* to express tumor-specific antigen receptors, and administered (e.g., by adoptive transfer) to a mammal. Adoptively transferred naïve T cells can migrate to one or more lymph nodes to be activated *in vivo.* In some cases, naive T cells can be engineered to express tumor-specific antigen receptors *in situ.* For example, expression vectors (e.g., viral vectors) can be injected into secondary lymphoid organs such that naive T cells are engineered *in situ* to express tumor-specific antigen receptors. When the naïve T cells expressing tumor-specific antigen receptors encounter an antigen (e.g., an antigen presented by an APC such as a subcapsular sinus macrophage and/or a dendritic cell), the naive T cells are activated (e.g., to become CTLs) *in vivo.* The *in vivo* activated T cells can target cells (e.g., cancer cells) expressing the antigen (e.g., a tumor antigen) recognized by the tumor-specific antigen receptors. In some cases, the *in vivo* activated T cells can target cancer cells in tissues that lack current and/or preexisting inflammation. In some cases, the *in vivo* activated T cells do not target normal (e.g., healthy a non-cancerous) cells.

A naive T cell that can be activated *in vivo* as described herein can be any appropriate naive T cell. Examples of naive T cells include, without limitation, CTLs (e.g., CD4+ CTLs and/or CD8+ CTLs). For example, a naive T cell that can be activated *in vivo* as described herein can be a CD8+ CTL. In some cases, one or more naive T cells can be obtained from a mammal (e.g., a mammal having cancer). For example, naive T cells can be obtained from a mammal to be treated with the materials and method described herein.

A naive T cell activated *in vivo* as described herein can express (e.g., can be engineered to express) any appropriate antigen receptor. In some cases, an antigen receptor can be a heterologous antigen receptor. In some cases, an antigen receptor can be a chimeric antigen receptor (CAR). In some cases, an antigen receptor can be a tumor antigen (e.g., tumor-specific antigen) receptor. For example, a naive T cell can be engineered to express a tumor-specific antigen receptor that targets a tumor antigen (e.g., a cell surface tumor antigen) expressed by a cancer cell in a mammal having cancer. In some cases, an antigen receptor can be an indirect antigen receptor. For example, a naive T cell can be engineered to express an indirect antigen receptor that targets a first antigen (e.g., an exogenous antigen). In some cases, a target cell (e.g., a cancer cell in a mammal having cancer) can express a first antigen (e.g., a tumor antigen) can be recognized by a reagent (e.g., an antibody) containing a second antigen, and a naive T cell can be engineered to express an antigen receptor that targets the second antigen. In some cases, a tumor antigen can be a tumor-specific antigen (TSA; e.g., a tumor antigen present only on tumor cells). In some cases, a tumor antigen can be a tumor-associated antigen (TAA; e.g., an abnormal protein present on tumor cells). Examples of tumor antigens that can be recognized by a tumor antigen receptor expressed in a naïve T cell include, without limitation, mucin 1 (MUC-1), human epidermal growth factor receptor 2 (HER-2), estrogen receptor (ER), epidermal growth factor receptor (EGFR), folate receptor alpha, and mesothelin. As described herein, a naive T cell can be engineered to have an antigen receptor (e.g., a heterologous antigen receptor) that recognizes any appropriate antigen. In some cases, a naive T cell can be engineered to have an antigen receptor (e.g., a heterologous antigen receptor) that recognizes persistent virus antigens or senescent cells.

Any appropriate method can be used to express an antigen receptor on a naive T cell. For example, a nucleic acid encoding an antigen receptor can be introduced into the one or more naive T cells. In some cases, viral transduction can be used to introduce a nucleic acid encoding an antigen receptor into a non-dividing cell. A nucleic acid encoding an antigen receptor can be introduced in a naive T cell using any appropriate method. In some cases, a nucleic acid encoding an antigen receptor can be introduced into a naive T cell by transduction (e.g., viral transduction using a retroviral vector or a lentiviral vector) or transfection. In some cases, a nucleic acid encoding an antigen receptor can be introduced *ex vivo* into one or more naive T cells. For example, *ex vivo* engineering of naive T cells expressing an antigen receptor can include transducing isolated naive T cells with a lentiviral vector encoding an antigen receptor. In cases where naive T cells are engineered *ex vivo* to express an antigen receptor, the naive T cells can be obtained from any appropriate source (e.g., a mammal such as the mammal to be treated or a donor mammal, or a cell line). In some cases, a nucleic acid encoding an antigen receptor can be introduced into one or more naïve T cells *in situ* into the lymphatic system (e.g., into one or more secondary lymphoid organs such as the lymph nodes and the spleen). For example, *in situ* engineering of naïve T cells to express an antigen receptor can include intradermal (ID) injection (e.g., directly into one or more lymph nodes) of a lentiviral vector encoding an antigen receptor.

Any appropriate method can be used to activate the naive T cells described herein (e.g., engineered naive T cells such as naive T cells designed to express tumor-specific antigen receptors). For example, naive T cells expressing tumor-specific antigen receptors can be activated *in vivo* by administering one or more immunogens (e.g., antigens) to a mammal. Any appropriate immunogen can be used to activate a naive T cell described herein. In some cases, an immunogen can be a cell surface antigen (e.g., a cell surface antigen expressed by a cancer cell). In some cases, an immunogen can be an allogeneic immunogen (e.g., an allogeneic antigen (also referred to as an alloantigen)). Examples of antigens that can be used to activate the naive T cells described herein include, without limitation, an allogeneic MHC class I polypeptide (allo-MHC I or alloMHC I polypeptide) and an allogeneic MHC class II polypeptide (allo-MHC II or alloMHC II polypeptide). Such antigens can be presented as one or more fragments in the context of an MHC molecule such as MHC I. For example, naive T cells expressing tumor-specific antigen receptors can be activated *in vivo* by administering allo-MHC I to a mammal.

Any appropriate method can be used to administer an immunogen (e.g., an antigen) to a mammal (e.g., a human). Examples of methods of administering immunogens to a mammal can include, without limitation, injections (e.g., intravenous (IV), ID, intramuscular (IM) injection, or subcutaneous). In some cases, an antigen can be encoded by a vector (e.g., a viral vector), and the vector can be administered to a mammal.

An exemplary nucleic acid sequence encoding a human allo-MHC I can include a sequence as set forth in SEQ ID NO: 1. Nucleic acid encoding a human MHC I polypeptide (e.g., an HLA-A polypeptide, an HLA-B polypeptide, or an HLA-C polypeptide) can be included within a viral vector such that cells infected with the viral vector express the encoded MHC I polypeptide. In some cases, a nucleic acid sequence encoding a human allo-MHC I can be as described elsewhere (see, e.g., Pimtanothai et al., 2000 Human Immunology 61:808-815). In some cases, a nucleic acid sequence encoding a human allo-MHC I can be as set forth in a database such as the National Center for Biotechnology Information (see, e.g., GenBank^{®} accession numbers M84384.1, AF181842, and AF181843).

SEQ ID NO:1
ATGCGGGTCACGGCGCCCCGAACCCTCCTCCTGCTGCTCTGGGGGGCAGTGGCCCTGACC
GAGACCTGGGCTGGCTCCCACTCCATGAGGTATTTCCACACCTCCGTGTCCCGGCCCGGC
CGCGGGGAGCCCCGCTTCATCACCGTGGGCTACGTGGACGACACGCTGTTCGTGAGGTTC
GACAGCGACGCCACGAGTCCGAGGAAGGAGCCGCGGGCGCCATGGATAGAGCAGGAGGGG
CCGGAGTATTGGGACCGGGAGACACAGATCTCCAAGACCAACACACAGACTTACCGAGAG
AGCCTGCGGAACCTGCGCGGCTACTACAACCAGAGCGAGGCCGGGTCTCACATCATCCAG
AGGATGTATGGCTGCGACCTGGGGCCGGACGGGCGCCTCCTCCGCGGGCATAACCAGTAC

GCCTACGACGGCAAAGATTACATCGCCCTGAACGAGGACCTGAGCTCCTGGACCGCGGCG
GACACCGCGGCTCAGATCACCCAGCGCAAGTGGGAGGCGGCCCGTGAGGCGGAGCAGCTG
AGAGCCTACCTGGAGGGCCTGTGCGTGGAGTGGCTCCGCAGACACCTGGAGAACGGGAAG
GAGACGCTGCAGCGCGCGGACCCCCCAAAGACACACGTGACCCACCACCCCATCTCTGAC
CATGAGGCCACCCTGAGGTGCTGGGCCCTGGGCTTCTACCCTGCGGAGATCACACTGACC
TGGCAGCGGGATGGCGAGGACCAAACTCAGGACACTGA

An exemplary nucleic acid sequence encoding a human allo-MHC II can include a sequence as set forth in SEQ ID NO:2. Nucleic acid encoding a human MHC II polypeptide (e.g., an HLA-DP polypeptide, an HLA-DM polypeptide, an HLA-DOA polypeptide, an HLA-DOB polypeptide, an HLA-DQ polypeptide, or an HLA-DR polypeptide) can be included within a viral vector such that cells infected with the viral vector express the encoded MHC II polypeptide. In some cases, a nucleic acid sequence encoding a human allo-MHC II can be as described elsewhere (see, e.g., Robinson et al., 2005 Nucleic Acids Research 331:D523-526; and Robinson et al., 2013 Nucleic Acids Research 41:D1234-40).

SEQ ID NO:2
ATGGTGTGTCTGAGGCTCCCTGGAGGCTCCTGCATGGCAGTTCTGACAGTGACACTGATG
GTGCTGAGCTCCCCACTGGCTTTGGCTGGGGACACCAGACCACGTTTCTTGGAGTACTCT
ACGGGTGAGTGTTATTTCTTCAATGGGACGGAGCGGGTGCGGTTACTGGAGAGACACTTC
CATAACCAGGAGGAGCTCCTGCGCTTCGACAGCGACGTGGGGGAGTTCCGGGCGGTGACG
GAGCTGGGGCGGCCTGTCGCCGAGTCCTGGAACAGCCAGAAGGACATCCTGGAAGACAGG
CGCGCCGCGGTGGACACCTATTGCAGACACAACTACGGGGCTGTGGAGAGCTTCACAGTG
CAGCGGCGAGTCCATCCTAAGGTGACTGTGTATCCTTCAAAGACCCAGCCCCTGCAGCAC
CACAACCTCCTGGTCTGTTCTGTGAGTGGTTTCTATCCAGGCAGCATTGAAGTCAGGTGG
TTCCGGAATGGCCAGGAAGAGAAGACTGGGGTGGTGTCCACGGGCCTGATCCACAATGGA
GACTGGACCTTCCAGACCCTGGTGATGCTGGAAACAGTTCCTCGGAGTGGAGAGGTTTAC
ACCTGCCAAGTGGAGCACCCAAGCGTGACAAGCCCTCTCACAGTGGAATGGAGAGCACGG
TCTGAATCTGCACAGAGCAAGATGCTGAGTGGAGTCGGGGGCTTTGTGCTGGGCCTGCTC
TTCCTTGGGGCCGGGCTGTTCATCTACTTCAGGAATCAGAAAGGACACTCTGGACTTCAG
CCAAGAGGATTCCTGAGCTGA

In some cases, a nucleic acid set forth in Figure 17 can be included within a viral vector to express a human MHC I polypeptide, and that viral vector can be used to active naive T cells within a mammal.

In some cases, a viral vector for activating naive T cells *in vivo* as described herein can be designed to express a fragment of an MHC I polypeptide or a fragment of an MHC II polypeptide. A fragment of an MHC I polypeptide or an MHC II polypeptide can be from about 182 amino acids to about 273 amino acids (e.g., from about 182 amino acids to about 250 amino acids, from about 182 amino acids to about 225 amino acids, from about 182 amino acids to about 200 amino acids, from about 200 amino acids to about 273 amino acids, from about 225 amino acids to about 273 amino acids, from about 250 amino acids to about 273 amino acids, from about 190 amino acids to about 260 amino acids, from about 200 amino acids to about 250 amino acids, from about 215 amino acids to about 235 amino acids, from about 200 amino acids to about 220 amino acids, from about 220 amino acids to about 240 amino acids, from about 240 amino acids to about 260 amino acids, or from about 260 amino acids to about 280 amino acids) in length.

A viral vector for activating naive T cells *in vivo* as described herein can be, or can be derived from, a viral vaccine. In some cases, a viral vector used as described herein can be replication-defective. In some cases, a viral vector used as described herein can be immunogenic. Examples of viral vectors that can be designed to encode an MHC class I or class II polypeptide and used to active T cells (e.g., naive T cells) within a mammal include, without limitation, picornavirus vaccines, adenovirus vaccines, rhabdoviruses (e.g., vesicular stomatitis viruses (VSV)), paramyxoviruses, and lentiviruses. In some cases, naive T cells described herein can be activated *in vivo* by administering to a human an immunogenic, replication-defective adenoviral vector encoding an allo-MHC I. An exemplary adenoviral vector encoding an allo-MHC I and/or allo-MHC-class II is shown in Figure 4B.

This document also provides materials for use in methods for treating mammals (e.g., humans) having cancer (e.g., a cancer including cancer cells that express a tumor antigen). For example, naive T cells described herein (e.g., naive T cells expressing a tumor-specific antigen) can be activated *in vivo* for use in methods for treating humans having cancer. In some cases, *in vivo* activation of naive T cells as described herein can be used for use in a method to reduce the number of cancer cells (e.g., cancer cells expressing a tumor antigen) within a mammal. In some cases, *in vivo* activation of naive T cells as described herein can be used in a method to slow and/or prevent recurrence of a cancer (e.g., a cancer in remission). In some cases, *in vivo* activation of naive T cells as described herein can be used in a method to target quiescent and/or non-dividing cancer cells (e.g., cancer cells expressing tumor antigens).

In some cases, the materials described herein can be used in a method of treating mammals (e.g., humans) having cancer where the method includes identifying the mammal as having cancer. Any appropriate method can be used to identify a mammal as having cancer. Once identified as having cancer, naive T cells (e.g., naive T cells obtained from the mammal having cancer) can be engineered (e.g., engineered *in vitro* or *in vivo)* to express antigen receptors (e.g., tumor-specific antigen receptors), and activated *in vivo* as described herein.

The materials described herein can be used in methods of treating cancer in any type of mammal having cancer, for example, without limitation, primates (e.g., humans and monkeys), dogs, cats, horses, cows, pigs, sheep, rabbits, mice, and rats. For example, the materials described herein can be used in methods of treating humans having cancer using *in vivo* activation of naïve T cells as described herein.

The materials described herein can be used in methods of treating any appropriate type of cancer. In some cases, a cancer to be treated as described herein can include one or more solid tumors. In some cases, a cancer to be treated as described herein can be a cancer in remission. In some cases, a cancer to be treated as described herein can include quiescent (e.g., dormant or non-dividing) cancer cells. In some cases, a cancer to be treated as described herein can be cancer that has escaped and/or has been non-responsive to chemotherapy. Examples of cancers that can be treated by *in vivo* activation of naive T cells as described herein include, without limitation, leukemias (e.g., acute lymphoblastic leukemia (ALL), acute myelogenous leukemia (AML), chronic lymphocytic leukemia (CLL), small lymphocytic lymphoma (SLL), chronic myelogenous leukemia (CML), acute monocytic leukemia (AMOL)), lymphomas (e.g., Hodgkin's lymphomas and non-Hodgkin's lymphomas), myelomas, ovarian cancer, breast cancer, prostate cancer, colon cancer, germ cell tumors, hepatocellular carcinoma, bowel cancer, lung cancer, and melanoma (e.g., malignant melanoma).

The materials and methods described herein can be used to specifically target a cell (e.g., a cancer cell) expressing an antigen (e.g., a tumor antigen such as a tumor-specific antigen). For example, *in vivo* activation of naive T cells as described herein can include engineering the naive T cells to express a tumor-specific antigen receptor that can target (e.g., recognize and bind to) a tumor antigen. In some cases, a tumor antigen can be a cell surface tumor antigen. Examples of tumor antigens that can be targeted by *in vivo* activated T cells expressing a tumor-specific antigen receptor include, without limitation, MUC-1 (associated with breast cancer, multiple myeloma, colorectal cancer, and pancreatic cancer), HER-2 (associated with gastric cancer, salivary duct carcinomas, breast cancer, testicular cancer, and esophageal cancer), and ER (associated with breast cancer, ovarian cancer, colon cancer, prostate cancer, and endometrial cancer).

In cases where naive T cells described herein (e.g., naïve T cells expressing tumor-specific antigen receptors) are engineered *ex vivo* to express a heterologous antigen receptor (e.g., a heterologous tumor-specific antigen receptor) as described herein and administered (e.g., by adoptive transfer) to a mammal (e.g., a human), any appropriate method can be used to administer the naive T cells (e.g., engineered naive T cells). Examples of methods of administering naive T cells engineered to express a heterologous antigen receptor to a mammal can include, without limitation, injection (e.g., IV, ID, IM, or subcutaneous injection). For example, naïve T cells expressing tumor-specific antigen receptors can be administered to a human by IV injection.

In cases where naive T cells described herein (e.g., naïve T cells expressing tumor-specific antigen receptors) are engineered *ex vivo* to express a heterologous antigen receptor (e.g., a heterologous tumor-specific antigen receptor) and administered (e.g., by adoptive transfer) to a mammal (e.g., a human), any appropriate number of naive T cells (e.g., engineered naive T cells) can be administered to a mammal (e.g., a mammal having cancer). In some cases, from about 200 naïve T cells described herein to about 1500 naive T cells described herein (e.g., from about 200 naive T cells to about 1300 naive T cells, from about 200 naive T cells to about 1250 naive T cells, from about 200 naive T cells to about 1000 naive T cells, from about 200 naive T cells to about 750 naive T cells, from about 200 naive T cells to about 500 naive T cells, or from about 200 naive T cells to about 400 naive T cells) can be administered to a mammal (e.g., a human). For example, about 300 naive T cells expressing tumor-specific antigen receptors can be administered to a human having cancer where the naive T cells are then activated *in vivo* by allo-MHC I (e.g., allo-MHC I administered to the human having cancer using an immunogenic, replication-defective adenoviral vector encoding an allo-MHC I).

The invention will be further described in the following examples, which do not limit the scope of the invention described in the claims.

### EXAMPLES

### Example 1: Priming Cytotoxic T Cells (CTLs).

To examine if CTLs could be primed to hunt and kill quiescent cells expressing targetable antigens, 1200 OT-1 T cells were adoptively transferred into RIP-OVA mice (expressing the ovalbumin (OVA) antigen in pancreatic islets), and then activated with TMEV-OVA picornavirus vaccine.

Pancreatic tissues were examined at using H&E staining and IHC staining for insulin. Pancreatic inflammation was seen within 5 days of CTL induction by virus (Figure 2A). Significant destruction of islets was observed in surviving mice on day 21 (Figure 2B). No virus was detected in the pancreas by PCR. As few as 300 naive T cells activated *in vivo* by a picornavirus vaccine elicited complete destruction of normal virus free pancreatic islets within 10 days of activation. In contrast, 8 X 10⁷ OT-1 spleen cells activated in donor mice and transferred into RIP-OVA mice were not pathogenic.

These results demonstrate that activated T cells can scan cells in the body for relevant antigens and elicit immune destruction in the absence of preexisting inflammation.

### Example 2: Activation of Allo-Reactive Cytotoxic T Cells (CTLs).

To determine whether adenovirus encoding allogeneic MHC I molecules can activate allo-reactive CTL, the allogeneic MHC class I gene was expressed in the context of an adenovirus infection into LN antigen presenting cells.

Adenovirus expressing Cre recombinase were introduced into the lymphatics of mTmG-reporter mice by intradermal injection. mTmG-reporter mice express a floxed membrane red fluorescent "tomato" and a silenced membrane GFP gene. In the presence of expressed cre, tomato is silenced and GFP is activated. Tomato expressing and GFP expressing T cells can be distinguished by fluorescent microscopy following introduction of an adenovirus expressing cre or a control adenovirus. Successful transduction results in the transition from red to green fluorescence. Cre recombinase was transduced in sub capsular sinus macrophage (Figures 3A - 3C).

A replication-defective adenovirus (serotype 6) vector expressing a mutant MHC molecule which functions as a universal alloantigen (Figure 4A) was introduced into LN by intradermal injection. Four days after introduction of adenovirus encoding allogeneic MHC I, syngeneic (BALB/c), allogeneic (B6), and third party (C3H) labeled target cells were adoptively transferred IV into challenged hosts in an *in vivo* CTL assay. Four hours later spleen cells were harvested and analyzed by flow cytometry for the presence of introduced target cells. B6 target cells (targets expressing introduced allo-MHC I) were completely eliminated *in vivo.* Potent allo-reactive CD8+ T cells were activated in just 4 days (Figure 5).

These results demonstrate that intradermally injected adenovirus expressing allo-MHC I can present allo-MHC I antigen in sub capsular sinus macrophages and can activate CTLs that target cells expressing allo-MHC I.

### Example 3: Adoptive Transfer of Naive Cytotoxic T Cells (CTLs).

To examine if adoptively transferred naive CTL precursors migrate to secondary lymphoid organs and become activated by adeno-MHCI virus, allotype-marked naive T cells were labeled with CFSE and adoptively transferred intravenously into naive hosts which were subsequently challenged intradermally with adeno-MHCI to elicit an allo-reactive CTL response from the transferred cells.

Adoptively transferred CFSE-labeled T cells migrated to the LN where they encountered and responded to transduced alloMHCI molecules (Figures 6A and 6C). Stimulated cells proliferated when stimulated with allo-MHCI, diluting the CFSE (Figure 6C). The CFSE-dilute population displayed a more activated phenotype expressing high CD44 and PD-1 (Figures 6D) relative to the CFSE-dilute cells isolated from lymph nodes challenged with control adenovirus (Figures 6B). Approximately 4.5% of the transferred cells present on day 4 had proliferated (Figures 6A and 6C) and exhibited upregulation of activation markers (Figures 6B and 6D).

These results demonstrate that adoptively transferred CD8+ T cells can migrate to the LN and can be activated by an alloMHC I encoding adenovirus vaccine.

### Example 4: In Vivo Activation of Naive Cytotoxic T Cells (CTLs).

To examine whether *in vivo* activated T cells can migrate into tumors, an approach for evaluating the efficiency of viral transduction of T cells *ex vivo* was established using mTmG-reporter mice. Naive CD8+ spleen cells from MTMG-reporter mice were transduced with lentivirus expressing cre by centrifugal concentration of virus and polybrene, and were subsequently activated with anti-CD3/CD28 + IL-2 to maintain viability in culture for 4 days. Successful transduction results in the transition from red to green fluorescence (Figure 7 and Figure 8). Using the MTMG-reporter mouse, the efficiency of transfection, the migration of adoptively transferred T cells into lymph nodes, and migration of the adoptively transferred T cells into tumors can be determined.

### Example 5: In Vivo Activation of Naive Cytotoxic T Cells (CTLs).

Humanized NSG (hu-NSG) mice with established human hematopoiesis provide a model for using lentivirus CAR to establish proof of concept. hu-NSG mice in donor matched batches with verified human leukocytes in circulation were obtained. These mice were used as donors of human cells for a CAR transduction scheme.

To determine if a CAR could activate CTLs *in vivo,* freshly isolated T cells were transduced with a lentiviral vector expressing human CAR19 (lenti-CAR19). Pooled CD4 & CD8 T cells transduced with lenti-CAR19 prior to or after activation (anti-CD3/CD28) and cultured 4 days to allow gene expression, then stained with anti-mouse antibody and analyzed using flow cytometry. Freshly isolated spleen cells were transduced with lenti-CAR19 for 1 hour and immediately transferred into syngeneic hu-NSG recipients (1 donor spleen/recipient). Mice also received Ad6-K^{b} vaccine at the time of cell transfer. Approximately 10% of the recovered human spleen cells were CAR+ in the three recipients. As shown in Figure 9, human T cells were effectively transduced with lento-CAR19, but mouse cells were not.

Hu-NSG mice lack lymph nodes. The absence of lymph nodes in hu-NSG mice required a change in approach. To evaluate the effectiveness of intradermal introduction of non-replicating virus in hu-NSG mice, Evans blue was injected intradermally in the tail to mark inguinal lymph node in WT, NOD Scid IL-2Rγ^{-/-} (NSG), and human CD34+ hematopoietic cell reconstituted NSG mice (hu-NSG) (Figure 10).

To determine if alternative routes of administration could be used for *in vivo* CTL, replication-defective adenoviral vectors encoding an allo-MHC I (Ad6-alloMHC (K^{b})) were delivered to hu-NSG mice multiple routes, and the ability to induce strong CTL activity was assessed. BALB/c mice received 10¹⁰ Ad6-K^{b} IV, ID, or IP. 1 week later, the mice received differentially labeled BALB/c (self) and B6 (alloMHC) target cells IV. Cells migrating into the spleen were assessed for both introduced populations. Effectiveness was indicated by the relative depletion of the B6 target cells. IV, ID, and IP vaccination were equally effective for inducing strong CTL activity (Figure 11). In a subsequent experiment, mice received half the vaccine IV and half IP as the distribution and trafficking of human immune cells in the spleens and peritoneum of NSG mice is poorly defined.

To determine whether adenovirus encoding allogeneic MHC I molecules can activate allo-reactive CTL to eliminated cells expressing a target antigen, hu-NSG mice were administered lentivirus-CAR19 transduced hu-NSG spleen cells and replication defective adenoviruses encoding the MHC allogeneic antigen H-2K^{b}. On overview of the method is shown in Figure 12. Three hu-NSG mice with known T cell reconstitution were selected as lymphoid donors. The human leukocyte composition of hu-NSG mice selected as donors and hu-NSG mice selected as recipients are shown in Figure 13. Spleen cells from donor animals were recovered, pooled, red cells lysed using ACK and then the whole product was suspended in 100 µL of undiluted lenti-CAR19 virus (MOI). Polybrene was added for final concentration of 8 µg/mL. The suspension was centrifuged at 800 x g for 90 minutes at 31 °C. The viral supernatant was removed, and the cell pellet was suspended in 300 µL PBS and injected IV (100 µL/mouse). 5 X 10⁹ viral particles of replication defective adenovirus 6 encoding the MHC allogeneic antigen H-2K^{b} was injected IV, and an identical dose was injected IP. The mice were monitored daily with no detrimental phenotypes observed for one week. On day 7, the mice were bled, and the composition of human B cells in the blood was assessed.

To determine if *in vivo* CTL induced anti-K^{b} cytotoxic activity in hu-NSG hosts, mice were challenged with a mixture of K^{b-} and K^{b+} target cells, and spleens of the recipient mice were examined. Four hours prior to harvesting blood and spleen cells from the hu-NSG recipients (which had received lentivirus-CAR19 transduced hu-NSG spleen cells and Ad6-H-2K^{b} vaccine 1 week earlier), mice were challenged with a 1:1 mixture of K^{b-} syngeneic NOD splenic target cells and K^{b+} allogeneic B6 splenic target cells differentially labeled with CFSE. Following the 4 hour *in vivo* incubation period, each of the spleens of the recipient mice was examined for the ratio of persisting labeled K^{b-} and K^{b+} target cells. The expected 1:1 ratio was altered in all three recipients indicating a preferential killing of the K^{b+} spleen cells (Figure 14, panel A). The ratio of recovered K^{b+} cells was significantly lower relative to the K^{b-} target cells (Figure 14, panel B). This analysis indicated CTL activity was induced by vaccination with Ad6-H-2K^{b} in the hu-NSG mice targeting K^{b} expressing cells.

To determine if *in vivo* CTL activated against cells expressing a target antigen, human spleen cells from hu-NSG hosts that received CART treatment and AD6 vaccination were assessed for expression of the CAR19 protein. Raw data of the drop in B cell numbers in hu-NSG mice is shown in Figure 15. The absolute numbers of recovered B cells pre and post therapy were highly significant. However, two variables could have contributed to this conclusion, non-specific depletion of peripheral blood cell populations, including B cells, by repeated blood sampling, and the intended effects of CAR T cell therapy. To confirm the drop in B cell numbers was due to CAR T cell therapy, data was normalized to remove possible non-specific depletion effects. The normalization of the post treatment values to the pretreatment values using the formula (total CD45+ cell counts pretreatment/CD45 cell counts post treatment X absolute counts of cell lineage+ cells post treatment) was a conservative approach, reducing the magnitude of observed differences between pre and post treatment values in the B cell compartment to account for no-specific depletion of B cells by repetitive sampling of the blood. One-tailed hypothesis testing used a paired T Test to reflect the hypothesis. The apparent increase in T cells following therapy is consistent with previous CART therapy findings. However, evaluation of this possibility was not part of the original hypothesis, therefore, a two tailed test was applied. The absence of change in myeloid counts suggests the observed drop in B cells and the apparent rise of T cells appears to be cell lineage specific (Figure 16). There appears to be a correlation between the degree of B cell depletion and rise in T cells and in the level of CAR19 expression (Figure 16, panel B) in the spleens of the recipient mice, associations also seen previously in CART therapy.

This analysis verified Ad6-K^{b} activated antigen-specific killing. The mice demonstrated activity against the CD19 target after administration of Ad6-MHC, as demonstrated by the depletion of circulating CD19+ B cells in the recipient mice.

These results demonstrate that naive T cells expressing tumor-specific antigen receptors can be specifically activated (e.g., to become CTLs) *in vivo* by encountering a target antigen, and the *in vivo* activated T cells can target cells expressing the antigen.

### Example 6: Generation of Viral Vectors.

To develop a viral vector encoding rare HLA class I molecules such as HLA-B*4028, partial nucleic acid sequences encoding exons 2 and 3 were obtained from publically available database (see, e.g., GenBank: AF181842 and AF181843, respectively; since replaced with AH008245.2) and were used to guide modification of the full-length coding sequence for HLA-B*4004 (see, e.g., GenBank: M84384.1) capable of producing a full-length HLA-B*4028 polypeptide (e.g., SEQ ID NO:3). To develop a viral vector also encoding rare HLA class II molecules such as HLA-DRB1*12:01:01:01, SEQ ID NO:2 was obtained from publically available data base (see, e.g., Robinson et al., 2005 Nucleic Acids Research 331:D523-526; and Robinson et al., 2013 Nucleic Acids Research 41:D1234-40), and used to produce a full-length HLA-DRB1*12:01:01:01 polypeptide (e.g., SEQ ID NO:4).

### OTHER EMBODIMENTS

It is to be understood that while the invention has been described in conjunction with the detailed description thereof, the foregoing description is intended to illustrate and not limit the scope of the invention, which is defined by the scope of the appended claims.

## Claims

1. A nucleic acid encoding a heterologous antigen receptor, wherein said heterologous antigen receptor is a tumor-specific antigen receptor; and
a replication-defective adenovirus comprising nucleic acid encoding an allogeneic MHC class I polypeptide, wherein said allogeneic MHC class I polypeptide is an HLA-A, HLA-B, or HLA-C polypeptide for use in a method for obtaining an activated T cell to target cancer cells within a mammal and treating cancer within said mammal, wherein said activated T cell comprises said heterologous antigen receptor, wherein said method comprises:
administering, to a mammal, said nucleic acid and said adenovirus, wherein said nucleic acid is administered to said mammal after said adenovirus is administered to said mammal, and wherein said nucleic acid is administered to said mammal via injection into a lymph node of said mammal,
activating T cells within said mammal, wherein administration of said adenovirus activates T cells within said mammal, wherein said adenovirus is administered to said mammal via intradermal injection, and
introducing said nucleic acid into T cells within said mammal to form engineered T cells comprising said heterologous antigen receptor, wherein said nucleic acid encoding said heterologous antigen receptor is introduced into said T cells with a viral vector comprising said nucleic acid, wherein said viral vector is a lentiviral vector or retroviral vector, wherein at least one T cell within said mammal comprises said heterologous antigen receptor and is activated to target said cancer cells.

2. The nucleic acid and the adenovirus for use of claim 1, wherein said mammal is a human, and/or wherein said at least one T cell is a cytotoxic T lymphocyte, and/or wherein said antigen receptor is a chimeric antigen receptor.

3. The nucleic acid and the adenovirus for use of any one of claims 1-2, wherein said adenovirus is administered to said mammal via direct administration into a lymph node of said mammal.

## Patentansprüche

1. Nukleinsäure, codierend einen heterologen Antigenrezeptor, wobei es sich bei dem heterologen Antigenrezeptor um einen tumorspezifischen Antigenrezeptor handelt; und
replikationsdefektes Adenovirus, umfassend Nukleinsäure, die ein allogenes MHC-Klasse-I-Polypeptid codiert, wobei es sich bei dem allogenen MHC-Klasse-I-Polypeptid um ein HLA-A-, HLA-B- oder HLA-C-Polypeptid handelt, zur Verwendung bei einem Verfahren zur Gewinnung einer aktivierten T-Zelle, die auf Krebszellen in einem Säuger zielt, und zur Behandlung von Krebs in dem Säuger, wobei die aktivierte T-Zelle den heterologen Antigenrezeptor umfasst, wobei das Verfahren Folgendes umfasst:
Verabreichen der Nukleinsäure und des Adenovirus an einen Säuger, wobei die Nukleinsäure dem Säuger nach dem Verabreichen des Adenovirus an den Säuger verabreicht wird und wobei die Nukleinsäure dem Säuger über Injektion in einen Lymphknoten des Säugers verabreicht wird,
Aktivieren von T-Zellen in dem Säuger, wobei durch Verabreichung des Adenovirus T-Zellen im Säuger aktiviert werden, wobei das Adenovirus dem Säuger über intradermale Injektion verabreicht wird, und
Einführen der Nukleinsäure in T-Zellen in dem Säuger, so dass konstruierte T-Zellen gebildet werden, die den heterologen Antigenrezeptor umfassen, wobei die Nukleinsäure, die den heterologen Antigenrezeptor codiert, mit einem die Nukleinsäure umfassenden viralen Vektor in die T-Zellen eingeführt wird, wobei es sich bei dem viralen Vektor um einen Lentivirus-Vektor oder Retrovirus-Vektor handelt, wobei mindestens eine T-Zelle im Säuger den heterologen Antigenrezeptor umfasst und aktiviert wird, so dass sie auf die Krebszellen zielt.

2. Nukleinsäure und Adenovirus zur Verwendung nach Anspruch 1, wobei es sich bei dem Säuger um einen Menschen handelt und/oder wobei es sich bei der mindestens einen T-Zelle um einen zytotoxischen T-Lymphozyten handelt und/oder wobei es sich bei dem Antigenrezeptor um einen chimären Antigenrezeptor handelt.

3. Nukleinsäure und Adenovirus zur Verwendung nach einem der Ansprüche 1-2, wobei das Adenovirus dem Säuger über direkte Verabreichung in einen Lymphknoten des Säugers verabreicht wird.

## Revendications

1. Acide nucléique codant pour un récepteur d'antigène hétérologue, dans lequel ledit récepteur d'antigène hétérologue est un récepteur d'antigène spécifique d'une tumeur ; et
un adénovirus à réplication défectueuse comprenant un acide nucléique codant pour un polypeptide MHC allogénique de classe I, dans lequel ledit polypeptide MHC allogénique de classe I est un polypeptide HLA-A, HLA-B ou HLA-C destiné à être utilisé dans un procédé d'obtention d'une cellule T activée pour cibler des cellules cancéreuses chez un mammifère et de traitement d'un cancer chez ledit mammifère, où ladite cellule T activée comprend ledit récepteur d'antigène hétérologue, où ledit procédé comprend :
l'administration, à un mammifère, dudit acide nucléique et dudit adénovirus, où ledit acide nucléique est administré audit mammifère après l'administration dudit adénovirus audit mammifère, et où ledit acide nucléique est administré audit mammifère par injection dans un ganglion lymphatique dudit mammifère,
l'activation de cellules T dans ledit mammifère, dans lequel l'administration dudit adénovirus active des cellules T dans ledit mammifère, dans lequel ledit adénovirus est administré audit mammifère par injection intradermique, et
l'introduction dudit acide nucléique dans des cellules T à l'intérieur dudit mammifère pour former des cellules T modifiées comprenant ledit récepteur d'antigène hétérologue, dans lequel ledit acide nucléique codant pour ledit récepteur d'antigène hétérologue est introduit dans lesdites cellules T avec un vecteur viral comprenant ledit acide nucléique, ledit vecteur viral étant un vecteur lentiviral ou un vecteur rétroviral, dans lequel au moins une cellule T à l'intérieur dudit mammifère comprend ledit récepteur d'antigène hétérologue et est activé pour cibler lesdites cellules cancéreuses.

2. Acide nucléique et adénovirus pour utilisation selon la revendication 1, dans lesquels ledit mammifère est un humain, et/ou dans lesquels ladite au moins une cellule T est un lymphocyte T cytotoxique, et/ou dans lesquels ledit récepteur d'antigène est un récepteur d'antigène chimérique.

3. Acide nucléique et adénovirus pour utilisation selon l'une quelconque des revendications 1 et 2, dans lesquels ledit adénovirus est administré audit mammifère par administration directe dans un ganglion lymphatique dudit mammifère.
